Europäisches Patentamt

European Patent Office

Office européen des brevets

(11) Veröffentlichungsnummer: **0 007 103 B1**

(12) **EUROPÄISCHE PATENTSCHRIFT**

(45) Veröffentlichungstag der Patentschrift:
31.03.82

(51) Int. Cl.³: **A 61 B 17/36**

(21) Anmeldenummer: 79102409.4

(22) Anmeldetag: 12.07.79

(54) Kryochirurgiegerät.

(30) Priorität: 15.07.78 DE 2831199

(43) Veröffentlichungstag der Anmeldung:
23.01.80 Patentblatt 80/2

(45) Bekanntmachung des Hinweises auf die Patenterteilung:
31.03.82 Patentblatt 82/13

(84) Benannte Vertragsstaaten:
**BE FR GB IT NL**

(56) Entgegenhaltungen:
**US-A-3 393 679**
**US-A-3 451 395**
**DE-C-1 541 099**

(73) Patentinhaber: Erbe Elektromedizin GmbH. & Co.,KG,
Postfach 1420, D-7400 Tübingen (DE)

(72) Erfinder: van Gerven, Hans, Philosophenweg 45,
D-7400 Tübingen (DE)

(74) Vertreter: Endlich, Fritz, Dipl.-Phys., Postfach,
D-8034 Germering (DE)

Kryochirurgiegerät

Die Erfindung betrifft ein Kryochirurgiegerät mit einer Sonde zum Kühlen begrenzter Zonen von biologischen Geweben, deren thermisch gut leitende Spitze einen hohlen inneren Abschnitt aufweist, in den eine an eine Anschlußleitung für Druckgas anschließbare Zuführleitung mit einem verengten Austrittsspalt mündet, durch die das Druckgas zur Kühlung der Spitze aufgrund des Joule-Thomson-Effekts beim Durchfluß durch den Austrittsspalt zuführbar und durch eine zu der Atmosphäre hin offene koaxiale Auslaßleitung abführbar ist, welche in axialer Richtung relativ zu der sie umgebenden Zuführleitung verstellbar ist, sowie mit einem von der Außenseite her verstellbaren Ventil zur Steuerung der Arbeitstemperatur der Sondenspitze.

Bei einem bekannten Kryochirurgiegerät (US-A-3 393 679) ist ein normalerweise offenes Einlaßventil und ein normalerweise geschlossenes Auslaßventil vorgesehen, so daß durch gleichzeitiges Schließen des Einlaßventils bis auf eine enge Dosieröffnung und durch Öffnen des Auslaßventils mit Hilfe eines Handhebels flüssiges Kühlmittel einer durch die Sondenspitze gebildeten Verdampfungskammer zuführbar ist. Da das flüssige Kühlmittel normalerweise über einen Parallelspalt zugeführt wird und dieses Ventil im wesentlichen nur zwischen einer geöffneten und einer bis auf die enge Dosieröffnung geschlossenen Lage verstellt werden kann, ist dort eine genaue Steuerung der Arbeitstemperatur und eine Erzielung des Joule-Thomson-Effekts beim Durchfluß durch den Austrittsspalt nicht ohne weiteres möglich. Zur Erzielung einer schnellen Abkühlung ist es ferner erforderlich, eine Vakuumleitung an die Verdampfungskammer anzuschließen. Ein im Aufbau gleicher, jedoch in der Betriebsweise unterschiedliches Gerät ist aus der US-A-3 451 395 bekannt.

Kryochirurgiegeräte, bei denen die Sondenspitze mit Hilfe des Joule-Thomson-Effekts gekühlt wird, verwenden als Kühlmittel ein Gas, wie Lachgas ($N_2O$) oder Kohlendioxid ($CO_2$), wobei eine zusätzliche Vakuumleitung nicht erforderlich ist. Bei den meisten Kryochirurgiegeräten dieser Art ist es jedoch üblich, zur Temperatursteuerung im Bereich der Sondenspitze eine elektrische Heizeinrichtung anzuordnen, weil es in gewissen Fällen nicht ausreichend ist, durch Einstellung beispielsweise des Auslaßventils der Gasflasche die zugeführte Gasmenge so zu steuern, daß eine geeignete Temperatur der Sondenspitze erzielt werden kann (DE-C-1 541 099). Durch diese elektrische Heizeinrichtung kann zum Zwecke der Temperatursteuerung die durch den Joule-Thomson-Effekt erzielbare Kühlwirkung mehr oder weniger aufgehoben beziehungsweise kompensiert werden oder es kann nach dem Austritt aus dem verengten Spalt wieder eine Erwärmung erfolgen, um auf diese Weise eine geeignete Temperatur einzustellen.

Derartige Kryochirurgiegeräte, bei denen die Zuführleitung in der Sonde innerhalb der Auslaßleitung vorgesehen ist und bei denen am Ende der Zuführleitung eine Austrittsdüse angeordnet ist, haben sich in der Praxis zufriedenstellend bewährt. Andererseits wäre es jedoch zweckmäßiger, die innere der beiden koaxial angeordneten Leitungen als Auslaßleitung vorzusehen, damit das kalte Gas durch die innere Leitung zurückgeführt wird und durch das kalte Gas keine zusätzliche Abkühlung des Sondenschaftes verursacht wird. Bei einem bekannten Gerät dieser Art (DE-C-1 541 099) weist die innere Auslaßleitung an ihrem Ende einen in axialer Richtung einstellbaren äußeren Flansch auf, der einen solchen Außendurchmesser aufweist, daß zwischen dem Umfang des Flansches und der Innenfläche der Sonde ein sehr kleiner Abstand beziehungsweise eine Reihe von Abständen vorhanden sind, die durch Unregelmäßigkeiten des Flansches gebildet sind und eine Verengung darstellen. Die zur Steuerung der Temperatur erforderliche Heizwicklung kann dabei angrenzend an den Flansch der Auslaßleitung angeordnet werden. Abgesehen von dem Nachteil, daß derartige Geräte wegen der Heizwicklung eine Stromversorgung erfordern, ist es nicht ohne weiteres möglich, eine geeignete Steuerung der Temperatur zu erzielen, weil die thermische Ausdehnung beziehungsweise Kontraktion der den Spalt begrenzenden Elemente nicht ohne weiteres durch Stromzufuhr zu der Heizwicklung derart gesteuert werden können, daß eine geeignete Spaltgröße beibehalten werden kann.

Es ist deshalb Aufgabe der Erfindung, ein Kryochirurgiegerät der eingangs genannten Art unter möglichst weitgehender Vermeidung der genannten Nachteile und Schwierigkeiten derart zu verbessern, daß auf einfache Weise eine relativ genaue Steuerung der Arbeitstemperatur der Sondenspitze sowie eine Anpassung der Kühlleistung an verschiedene Sondengrößen und Sondenausführungen möglich ist.

Diese Aufgabe wird erfindungsgemäß dadurch gelöst, daß zur axialen Verstellung der Auslaßleitung eine Schraubverbindung zwischen der Auslaßleitung und der Zuführleitung vorgesehen ist, daß die Auslaßleitung zu ihrem den Austrittsspalt begrenzenden Ende hin sich außenseitig stärker konisch erweiternd als ein den Austrittsspalt begrenzender Bereich der Innenwand der Sonde ausgebildet ist und daß zum Abtauen der Sondenspitze die Auslaßleitung über eine Verbindungsleitung an die Anschlußleitung für das Druckgas anschließbar ist. Vorteilhafte Weiterbildungen der Erfindung sind Gegenstand der Unteransprüche.

Der Erfindung liegt die Erkenntnis zugrunde, daß es innerhalb des Dimensionierungsbereichs der zur Erzielung des Joule-Thomson-Effekts notwendigen Verengung möglich ist, den Aus-

2

trittsspalt während des Betriebs der Sonde derart nachzujustieren, daß thermische Ausdehnungen bzw. Kontraktionen oder durch den hohen Überdruck verursachte Deformationen kompensiert werden können und gleichzeitig eine Temperatursteuerung erfolgen kann. Die Herstellung einer derartigen Sonde kann in einfacherer und zuverlässigerer Weise erfolgen, weil bei der Herstellung keine engen Toleranzgrenzen für die Ausbildung des Austrittsspalts eingehalten werden müssen. Insbesondere ist es aber aus Sicherheitsgründen und aus konstruktiven Gründen vorteilhaft, daß in der Sonde keine Zuleitungen für eine elektrische Heizwicklung angeordnet werden müssen. Andererseits kann die Sondenspitze trotz des Fehlens der elektrischen Heizeinrichtung verhältnismäßig schnell aufgetaut werden, weil zu diesem Zweck der Sondenspitze über die Auslaßleitung auch das unter hohem Druck stehende Gas zugeführt werden kann, so daß die Temperatur der Sondenspitze durch Kondensation des Gases innerhalb weniger Sekunden ausreichend erhöht werden kann.

Anhand der Zeichnung soll die Erfindung beispielsweise näher erläutert werden. Es zeigt

Fig. 1 eine schematische Darstellung eines Kryochirurgiegeräts gemäß der Erfindung und

Fig. 2 einen Längsschnitt durch ein bevorzugtes Ausführungsbeispiel einer Sonde gemäß der Erfindung.

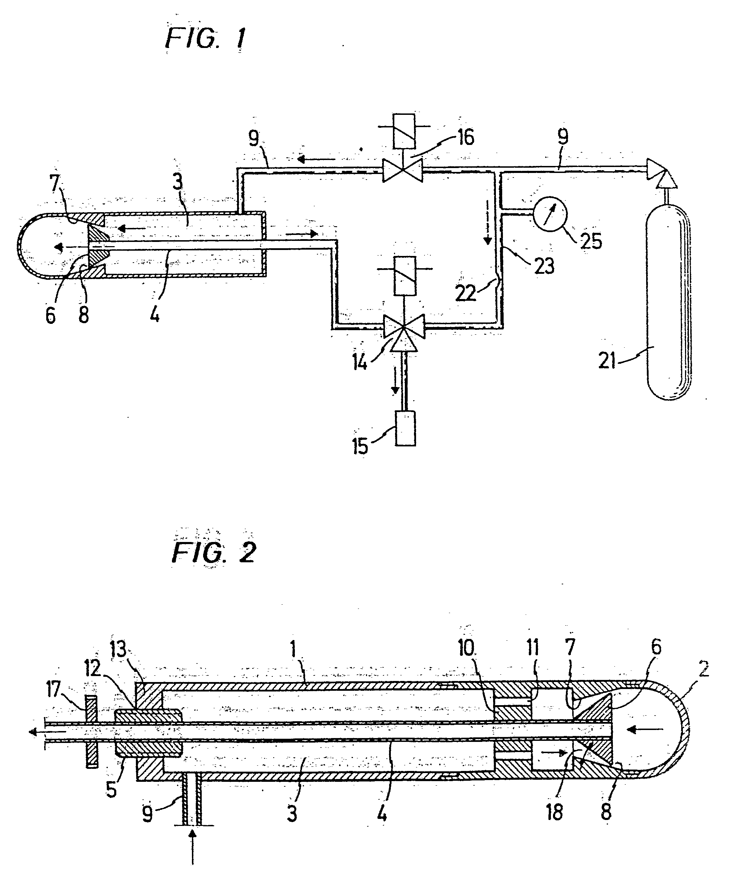

Die in Fig. 1 und 2 im Detail und vergrößert dargestellte Sonde 1 besitzt eine Sondenspitze 2, die vorzugsweise auswechselbar befestigt ist, so daß sie insbesondere durch eine Sondenspitze mit einer anderen Ausführungsform ersetzt werden kann, welche eine erheblich größere Kühlleistung erfordert.

Innerhalb einer Zuführleitung 3 ist eine Auslaßleitung 4 vorgesehen. Die Auslaßleitung 4 ist in axialer Richtung relativ zu der sie umgebenden Zuführleitung 3 verstellbar, weil im Bereich der der Sondenspitze 2 gegenüberliegenden Endwand 13 der Sonde eine Gewindemuffe 5 mit der Auslaßleitung 4 verbunden ist, die in einem Innengewinde 12 in der Endwand 13 angeordnet ist. Es ist jedoch auch möglich, im Bereich des äußeren Endes der Gewindemuffe 5 ein Innengewinde vorzusehen und die Auslaßleitung 4 mit einem entsprechenden Außengewinde zu versehen, wobei zweckmäßigerweise entlang dem Außenumfang in einer Verlängerungsbohrung der Gewindemuffe zwei Ringnuten für O-Ringe vorgesehen werden können, um eine Abdichtung gegenüber der Zuführleitung 3 zu gewährleisten. Angrenzend an das äußere Ende der Gewindemuffe 5 ist an der Auslaßleitung 4 ein gerändelter Verstellring 17 angeordnet.

An dem der Sondenspitze 2 benachbarten Ende der Auslaßleitung 4 weist die Auslaßleitung ein sich konisch erweiterndes Ende 6 auf, welches zusammen mit einem sich in Richtung auf die Sondenspitze 2 konisch erweiternden Bereich 7 der Innenwand der Sonde einen

Austrittsspalt 8 begrenzt, so daß die effektive Querschnittsfläche des Austrittsspalts 8 durch axiale Verschiebung der Auslaßleitung 4 verstellbar ist. Die Auslaßleitung 4 ist zwischen ihren gegenüberliegenden Enden in der Sonde 1 in einer Gasdurchtrittsöffnungen 11 aufweisenden Zentriereinrichtung 10 verschiebbar geführt.

An die Zuführleitung 3 ist eine Anschlußleitung 9 angeschlossen, die beispielsweise mit einer 8-kg-Flasche 21 (Fig. 1) in Verbindung steht, die $N_2O$ (Lachgas) oder $CO_2$ (Kohlendioxid) in gasförmigem Zustand unter einem Druck von mindestens 45 bar enthält. In der Anschlußleitung 9 ist ein Gaszuführventil 16 angeordnet.

An die Auslaßleitung 4 ist ein Mehrwegeventil 14 angeschlossen, durch welches das Gas über einen Schalldämpfer 15 ins Freie gelangen kann.

An die Anschlußleitung 9 ist ferner zwischen dem Ventil 16 und der Gasflasche 21 eine Leitung 23 angeschlossen, die mit dem Mehrwegeventil 14 über eine Drosselstelle 22 in Verbindung steht. An diese Leitung 23 ist ferner ein Manometer 25 angeschlossen.

Im folgenden soll die Arbeitsweise näher erläutert werden. Nach dem Öffnen des Ventils der Gasflasche 21 und dem Öffnen des Ventils 16 strömt das Gas durch die Anschlußleitung 9 und die Zuführleitung 3 durch den Austrittsspalt 8 in den Innenraum der Sondenspitze 2, um diese zu kühlen. Durch Verstellung der Effektivgröße des Austrittsspalts 8 durch Betätigung des Verstellrings 17 kann eine Steuerung der Sondentemperatur erforderlichenfalls erfolgen. Der Winkel 18 (Fig. 2) zwischen den konischen Oberflächen beträgt beispielsweise nur 4 bis 5°, so daß sich bei einem verhältnismäßig langen Bewegungsweg der Auslaßleitung 4 bei deren Verdrehung eine verhältnismäßig geringe Änderung der effektiven Querschnittsfläche des Spalts 8 ergibt. Aus diesem Grunde ist auch ein sehr feines Gewinde 12 vorgesehen, um eine empfindliche Steuerung bei dem Verdrehen des Verstellringes 17 zu ermöglichen.

Bei optimaler Einstellung des Austrittsspalts 8 ergibt sich die niedrigste erzielbare Kühltemperatur der Sondenspitze 2. Bei Vergrößerung des Austrittsspalts 8 und auch bei einer Verringerung des Austrittsspalts 8 ergibt sich eine Erhöhung der Temperatur der Sondenspitze 2 innerhalb eines Bereichs der Spaltgröße, die das Auftreten des Joule-Thomson-Effekts ermöglicht. Da die optimale Spaltgröße ohne weiteres einstellbar ist, können bei Vergrößerung der Spaltgröße erheblich größere Kühlleistungen erzielt werden, da in vielen Fällen eine gegenüber der minimalen Temperatur etwas erhöhte Temperatur in Kauf genommen werden kann. Deshalb können auswechselbare Sondenspitzen 2 in Verbindung mit der Sonde verwandt werden, die eine unterschiedliche Größe besitzen und beispielsweise bei einer erheblich größeren Oberfläche eine wesentlich größere Kühlleistung erfordern.

Zum Abtauen der Sondenspitze 2 wird das Mehrwegeventil 14 umgeschaltet, während das Ventil 16 geöffnet bleibt, so daß nunmehr das unter hohem Druck stehende Gas auch in Richtung des in Fig. 1 gestrichelt dargestellten Pfeils durch die Leitung 23 und die Auslaßleitung 4 in den Innenraum der Sondenspitze 2 gelangt, wodurch die Temperatur der auf Betriebstemperatur befindlichen Sondenspitze durch Kondensation des unter hohem Druck stehenden Gases an der Innenwand der Sondenspitze erhöht wird. Dadurch kann die Sondenspitze verhältnismäßig schnell auf ca. −10°C erwärmt werden, zumal die Sondenspitze eine verhältnismäßig geringe Wärmekapazität besitzt und auch eine Wärmezufuhr von der Außenseite her erfolgt. Durch eine elektronische Zeituhr wird das Mehrwegeventil 14 nach beispielsweise 10 Sekunden umgeschaltet und das Ventil 16 geschlossen, so daß dann die betreffenden Leitungen und die Sonde nicht mehr unter Überdruck stehen. Durch eine geeignete Ausbildung der Kupplung der Sonde mit dem Sondenschlauch ist es möglich, daß die Sonde nur im druckfreien Zustand von dem Sondenschlauch abgekuppelt werden kann.

Auch die Steuerung der Arbeitstemperatur der Sonde ist einfacher als eine Verwendung einer Heizwicklung zum Zwecke der Temperatursteuerung, weil nach der Durchführung einer geeigneten Einstellung des Austrittsspalts 8 auf die minimale Betriebstemperatur von beispielsweise etwa −80°C bei folgenden Benutzungen im allgemeinen keine Verstellung erforderlich ist, wenn sonst gleiche Bedingungen vorliegen. Wegen der Nachjustierbarkeit besteht auch eine größere Freiheit bei der Auswahl der verwendbaren Materialien, aus denen die Sonde hergestellt wird. Zweckmäßigerweise besteht jedoch das konische Ende 6 und auch der Bereich 7 der Innenwand aus einem Material mit dem gleichen thermischen Ausdehnungskoeffizienten. Das konische Ende 6 kann mit der Auslaßleitung 4 verlötet oder einstückig damit ausgebildet sein. Wie bereits erwähnt wurde, ist die Größe des Austrittsspalts 8 bei der Herstellung nicht kritisch und der sich vor dem Austrittsspalt 8 konisch verjüngende Bereich ist auch in strömungstechnischer Hinsicht günstiger als die Ausbildung eines radialen Flansches, weil durch Turbulenz in diesem Bereich Vibrationen der innen angeordneten Auslaßleitung verursacht werden könnten. Die Stabilität der Auslaßleitung 4 wird ferner durch die Anordnung der mit den Gasdurchtrittsöffnungen 11 versehenen Zentriereinrichtung 10 verbessert.

## Patentansprüche

1. Kryochirurgiegerät mit einer Sonde (1) zum Kühlen begrenzter Zonen von biologischen Geweben, deren thermisch gut leitende Spitze (2) einen hohlen inneren Abschnitt aufweist, in den eine an eine Anschlußleitung (9) für Druckgas anschließbare Zuführleitung (3) mit einem verengten Austrittsspalt (8) mündet, durch die das Druckgas zur Kühlung der Spitze (2) aufgrund des Joule-Thomson-Effekts beim Durchfluß durch den Austrittsspalt (8) zuführbar und durch eine zu der Atmosphäre hin offene koaxiale Auslaßleitung (4) abführbar ist, welche in axialer Richtung relativ zu der sie umgebenden Zuführleitung (3) verstellbar ist sowie mit einem von der Außenseite her verstellbaren Ventil zur Steuerung der Arbeitstemperatur der Sondenspitze (2), dadurch gekennzeichnet, daß zur axialen Verstellung der Auslaßleitung (4) eine Schraubverbindung (5, 12) zwischen der Auslaßleitung (4) und der Zuführleitung (3) vorgesehen ist, daß die Auslaßleitung (4) zu ihrem den Austrittsspalt (8) begrenzenden Ende (6) hin sich außenseitig stärker konisch erweiternd als ein den Austrittsspalt (8) begrenzender Bereich (7) der Innenwand der Sonde (1) ausgebildet ist und daß zum Abtauen der Sondenspitze (2) die Auslaßleitung (4) über eine Verbindungsleitung (23) an die Anschlußleitung (9) für das Druckgas anschließbar ist.

2. Kryochirurgiegerät nach Anspruch 1, dadurch gekennzeichnet, daß der Winkel (18) zwischen den den Austrittsspalt (8) bildenden konischen Oberflächen in durch deren gemeinsame Achse verlaufenden Ebenen kleiner als 5° ist.

3. Kryochirurgiegerät nach Anspruch 1 oder 2, dadurch gekennzeichnet, daß die beiden konischen Teile (6, 7) aus einem Material mit demselben thermischen Ausdehnungskoeffizienten bestehen.

4. Kryochirurgiegerät nach einem der vorhergehenden Ansprüche, dadurch gekennzeichnet, daß die Auslaßleitung (4) zwischen ihren gegenüberliegenden Enden in der Sonde (1) in einer Gasdurchtrittsöffnungen (11) aufweisenden Zentriereinrichtung (10) verschiebbar geführt ist.

5. Kryochirurgiegerät nach einem der vorhergehenden Ansprüche, dadurch gekennzeichnet, daß die Auslaßleitung (4) über ein Mehrwegeventil (14) zu der Atmosphäre hin offen ist, über das die Auslaßleitung (4) zum Abtauen der Sondenspitze (2) an die Anschlußleitung (9) an einer Stelle vor dem Gasführventil (16) mittels der eine Drosselstelle (22) enthaltenden Verbindungsleitung (23) anschließbar ist.

## Claims

1. A cryo-surgical instrument comprising a probe (1) for cooling restricted zones of biological tissues, the tip (2) of the probe being a good thermal conductor and having a hollow inner portion into which there leads a supply line (3) which is adapted to be connected to a connecting line (9) for pressure gas, has a restricted exit gap (8) and through which the pressure gas for cooling the tip (2) by the Joule-Thomson effect as it flows through the exit gap (8) is adapted to be supplied and is adapted to be discharged through a coaxial outlet line (4) open to atmosphere and displaceable axially relatively to its surrounding supply line (3), and comprising a valve adjustable from outside to control the working temperature of the probe tip (2), characterised in that a screw connection (5, 12) is provided between the outlet line (4) and the supply line (3) for axial adjustment of the outlet line (4), the outlet line (4) widens out conically towards its end (6) defining the exit gap (8), such conical widening being greater on the outside than an area (7) of the inner wall of the probe (1) bounding the exit gap (8), and for the purpose of defrosting the probe tip (2) the outlet line (4) is adapted to be connected to the pressure gas connecting line (9) via a connecting line (23).

2. A cryo-surgical instrument according to claim 1, characterised in that the angle (18) between the conical surfaces forming the exit gap (8) is less than 5° in planes extending through their common axis.

3. A cryo-surgical instrument according to claim 1 or 2, characterised in that the two conical parts (6, 7) consist of a material having the same coefficient of thermal expansion.

4. A cryo-surgical instrument according to any one of the preceding claims, characterised in that the outlet line (4) is displaceable between its opposite ends in the probe (1) in a centring device (10) formed with gas passage apertures (11).

5. A cryo-surgical instrument according to any one of the preceding claims, characterised in that the outlet line (4) is open to atmosphere via a multi-way valve (14) via which the outlet line (4) can be connected, in order to defrost the probe tip (2), to the connecting line (9) at a place upstream of the gas supply valve (16) by means of the connecting line (23) containing a throttle (22).

## Revendications

1. Instrument cryochirurgical comprenant une sonde (1) destinée à refroidir des zones restreintes de tissus biologiques, dont l'embout (2), bon conducteur de la chaleur, présente un tronçon intérieur creux où débouche un conduit (3) d'arrivée, pouvant être raccordé à un conduit (9) d'alimentation en gaz comprimé et ayant une fente (8) de sortie amincie par laquelle le gaz comprimé, destiné au refroidissement de l'embout (2) par effet Joule-Thomson, peut être amené et être évacué par un conduit (4) de sortie coaxial, ouvert sur l'atmosphère et pouvant être déplacé dans la direction axiale par rapport au conduit (3) d'arrivée qui l'entoure, ainsi qu'une soupape, pouvant être commandée de l'extérieur, permettant de maîtriser la température de travail de l'embout (2) de la sonde, caractérisé en ce que, pour le déplacement axial du conduit (4) de sortie, une liaison (5, 12) par vissage est interposée entre le conduit (4) de sortie et le conduit (3) d'arrivée, le conduit (4) de sortie s'évase coniquement vers l'extérieur plus fortement, vers son extrémité (6) délimitant la fente (8) de sortie, qu'une région (7), délimitant la fente (8) de sortie, de la paroi intérieure de la sonde (1) et, en vue de dégivrer l'embout (2) de la sonde, le conduit (4) de sortie peut être raccordé au conduit (9) d'alimentation en gaz comprimé par un conduit (23) de mise en communication.

2. Instrument cryochirurgical suivant la revendication 1, caractérisé en ce que l'angle (18) compris entre les surfaces coniques formant la fente (8) de sortie dans des plans passant par leur axe commun est inférieur à 5°.

3. Instrument cryochirurgical suivant la revendication 1 ou 2, caractérisé en ce que les deux parties (6, 7) coniques sont constituées d'une matière ayant le même coefficient de dilatation thermique.

4. Instrument cryochirurgical suivant l'une des revendications précédentes, caractérisé en ce que le conduit (4) de sortie est guidé entre ses extrémités opposées dans la sonde (1) de manière à pouvoir coulisser dans un dispositif (10) de centrage présentant des ouvertures (11) de passage pour le gaz.

5. Instrument cryochirurgical suivant l'une des revendications précédentes, caractérisé en ce que le conduit (4) de sortie est ouvert à l'atmosphère par l'intermédiaire d'une soupape (14) à plusieurs voies, par laquelle le conduit (4) de sortie peut être raccordée, en vue de dégivrer l'embout (2) de la sonde, au conduit (9) d'alimentation, en un endroit en amont de la soupape (16) pour l'amenée du gaz à l'aide du conduit (23) de mise en communication sur lequel est prévu un étranglement (22).

FIG. 1

FIG. 2